# DEMANDE DE BREVET EUROPEEN

(11) **EP 1 468 673 A1**
(43) Date de publication de la demande: **20.10.2004**
(21) Numéro de dépôt: 04290650.3
(22) Date de dépôt: 10.03.2004
(51) Int. Cl.: A61K 7/42

(54) **Emulsion eau dans huile contenant un tensioactif dérivé de polyoléfine et un 4,4-diarylbutadiene, utilisations**

(30) Priorité: 14.04.2003 FR 0304649
(71) Demandeur: L'OREAL, 75008 Paris (FR)
(72) Inventeur: L'Alloret, Florence, 75013 Paris (FR)
(74) Mandataire: Miszputen, Laurent

(57) **Abrégé**

L'invention concerne une émulsion eau-dans-huile comprenant au moins un tensio-actif polymérique constitué d'au moins une partie polaire et d'au moins une partie apolaire polyoléfinique, caractérisée par le fait qu'elle contient au moins un filtre UV-A organique du type 4,4-diarylbutadiène.

L'émulsion est stable dans le temps, présente une bonne efficacité photoprotectrice , une bonne rémanence à l'eau ainsi que de bonnes performances cosmétiques

## Description

L'invention concerne une émulsion eau-dans-huile comprenant au moins un tensio-actif polymérique constitué d'au moins une partie polaire et d'au moins une partie apolaire polyoléfinique, caractérisée par le fait qu'elle contient au moins un filtre UV-A organique du type 4,4-diarylbutadiène.

Il est bien connu que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

Les rayons UVA et UVB doivent donc être filtrés et il existe actuellement des compositions cosmétiques protectrices de l'épiderme humain renfermant des filtres UVA et UVB.

Parmi les formes galéniques à la disposition du formulateur, les émulsions eau dans huile sont particulièrement appréciées car elles peuvent former un film lipidique à la surface de la peau qui est ainsi protégée, ou encore assurer une bonne rémanence des filtres UV à la surface cutanée.

Les tensioactifs polymériques constitué d'au moins une partie polaire et d'au moins une partie apolaire polyoléfinique permettent de fabriquer des émulsions eau-dans-huile stables au stockage et adaptée aux applications cosmétiques, dermatologiques et/ou pharmaceutiques. En particulier, ils permettent de fabriquer des émulsions eau-dans-huile très riches en phase aqueuse (plus de 80%) et stables, ou bien des émulsions eau-dans-huile de faible viscosité et stables. Ces tensio-actifs présentent ainsi des avantages par rapport aux agents émulsionnants couramment utilisés pour stabiliser les émulsions eau dans huile, en particulier les alkyls polyglycérol, les alkyls POE, les alkyls de sorbitane, les sels métalliques d'acides gras et les tensioactifs siliconés. En effet pour ces émulsionnants, la teneur en phase aqueuse est en général inférieure à 80%, les concentrations en tensio-actifs sont élevées pour assurer une bonne stabilité des émulsions et la phase grasse comprend une proportion majoritaire d'huiles siliconées lorsque des tensio-actifs siliconés sont utilisés. D'autre part, les émulsions eau dans huile stabilisées par un tensio-actif dérivé de polyoléfines possèdent des propriétés cosmétiques particulièrement bonnes, avec un toucher léger et frais, sans être siliconé.

Parmi les filtres UV-A organiques disponibles, une famille de composés particulièrement efficaces dans l'UV-A est l'acide benzène 1,4-di(3-méthylidène-10-camphosulfonique) et ses différents sels, décrite notamment dans les demandes de brevets FR-A-2528420 et FR-A-2639347, ils sont capables en effet d'absorber les rayons ultraviolets de longueur d'ondes comprises entre 280 et 400 nm, avec des maxima d'absorption compris entre 320 et 400 nm, en particulier aux alentours de 345 nm.

Cependant, l'introduction de ce filtre UVA dans ces émuisions peut limiter leur stabilité, notamment leur tenue aux cycles de température, ou bien empêcher totalement la formation d'une émulsion lors de la fabrication.

Il apparaît ainsi nécessaire de disposer d'émulsions eau-dans-huile à base de tensio-actifs polymériques dérivés de polyoléfines stables pouvant contenir des filtres organiques actif dans l'UV-A d'efficacité comparable à celle de l'acide benzène 1,4-di(3-méthylidène-10-camphosulfonique) et ses différents sels sans les inconvénients énumérés ci-dessus.

La Demanderesse a découvert de façon surprenante que les émulsions eau-dans-huile comprenant au moins un tensio-actif polymérique dérivé de polyoléfine et au moins un filtre UV-A du type 4,4-diarylbutadiène, répondent à ce besoin.

Cette découverte est à la base de la présente invention.

Ainsi, conformément à l'un des objets de la présente invention, il est maintenant proposé une émulsion eau-dans-huile comprenant au moins un tensio-actif polymérique constitué d'au moins une partie polaire et d'au moins une partie apolaire polyoléfinique, caractérisée par le fait qu'elle contient au moins un filtre UV-A organique du type 4,4-diarylbutadiène.

D'autres caractéristiques, aspects et avantages de l'invention apparaîtront à la lecture de la description détaillée qui va suivre.

Par « émulsion stable », on entend au sens de la présente invention toute émulsion dont les aspects macroscopique et microscopique ne sont pas modifiés après 1 mois à la température ambiante.

Les tensioactifs polymériques de l'invention sont constitués d'au moins une partie polaire et d'au moins une partie apolaire polyoléfinique. Ils présentent en général une structure de type bloc ou peigne.

La partie apolaire polyoléfinique peut être choisie parmi les polymères et/ou copolymères d'éthylène, de propylène, de 1-butène, d'isobutène, de 1-pentène, de 2-méthyl-1-butène, de 3-methyl-1-butène, de 1-héxène, de 1-heptène, de 1-octène, de 1-décène, de 1-undécène, de 1-dodécéne, de 1-tridécène, de 1-tetradécène, de 1-pentadécène, de 1-hexadécéne, de 1-heptedécène et de 1-octadécéne. Les chaînes de polymères sont hydrogénées ou non. Elles sont constituées d'au moins 40 carbones, et de préférence de 60 à 700 carbones.

La partie polaire des oligomères ou polymères de l'invention peut être anionique, cationique, non ionique, zwitterionique ou amphotère. Elle est par exemple constituée de polyalkylène glycols ou de polyalkylène imines, ou encore d'acides ou de diacides carboxyliques, de leurs anhydrides ou de leurs dérivés, et leurs mélanges. Les oligomères ou polymères à partie polaire acide carboxylique peuvent être par exemple issus de la réaction entre une polyoléfine et au moins un acide ou anhydride carboxylique choisi dans le groupe comprenant l'acide maléique, l'anhydride maléique, l'acide fumarique, l'acide itaconique, l'acide citraconique, l'acide mésaconique, l'acide aconitique. De préférence, la partie polaire est constituée par l'acide ou l'anhydride succinique, leurs dérivés esters ou amides, les sels d'ions alcalins, alcalino-terreux ou organiques correspondants, ou bien encore par du polyoxyéthylène.
Les tensioactifs polymériques de l'invention sont par exemple des polymères diblocs polyisoprène-polyoxyéthylène ou poly(éthylène-co-propylène)-polyoxyéthylène décrits dans la publication de Allgaier, Poppe, Willner, Richter (Macromolecules, 1997, 30, p.1582-1586).

Les tensioactifs polymériques de l'invention sont également choisis parmi les polymères dérivés d'acide ou d'anhydride succinique décrits dans les brevets US-A-4,234,435, US-A-4,708,753, US-A-5,129,972, US-A-4,931,110, GB-A-2,156,799 et US-A-4,919,179. La partie polyoléfine peut être constituée par exemple de polyisobutylène, hydrogéné ou non, de poids moléculaire allant de 400 à 5000. Dans le polyisobutylène à terminaison succinique ainsi obtenu, la partie succinique peut être estérifiée, amidifiée ou sous forme de sel, c'est-à-dire qu'elle peut être modifiée par des alcools, des amines, des alcanolamines ou des polyols, ou encore se trouver sous forme de sels de métal alcalin ou alcalino-terreux, d'ammonium ou encore de base organique comme les sels de diéthanolamine et de triéthanolamine. Les polyoléfines à terminaison succinique estérifiée ou amidifiée sont des produits de réaction de (a) une polyoléfine à terminaison succinique, et de (b) une amine ou un alcool, pour former une amide ou un ester. Le terme « amine » utilisé ici comprend tous types d'amines dont les alcanolamines. Il peut s'agir par exemple de mono-amines primaires, secondaires ou tertiaires, ces amines pouvant être aliphatiques, cycloaliphatiques, aromatiques, hétérocycliques, saturées ou insaturées. Par ailleurs, les alcools peuvent être des mono- ou poly-alcools. Les mono-alcools comprennent les alcools aliphatiques primaires, secondaires ou tertiaires, et les phénols. Les poly-alcools peuvent être par exemple choisis parmi les poly-alcools aliphatique, cycloaliphatiques, aromatiques et hétérocycliques. Les polyoléfines à terminaison succinique modifiée (estérifiée ou amidifiée) et leur procédé de préparation sont décrits en particulier dans le document US-A-4,708,753.

Comme polyoléfines à terminaison succinique, on peut citer notamment les polyisobutylènes à terminaison succinique estérifiée et leurs sels, notamment les sels de diéthanolamine, tels que les produits commercialisés sous les dénominations Lubrizol 2724, Lubrizol 2722 et Lubrizol 5603 (Nom INCI: Hydroxyethyldiethonium polyisobutenyl triethylaminosuccinate (and) diethyl ethanolamine) par la société Lubrizol.

Un autre exemple de tensioactif polymérique utilisable dans l'invention est le produit de la réaction de l'anhydride maléique avec du polyisobutylène, tel que le Glissopal SA commercialisé par BASF.

Les tensio-actifs polyoléfiniques conformes à l'invention permettent en général d'abaisser la tension interfaciale (eau / huile) d'au moins 10 mN/m à une concentration de 0,01 % en poids par rapport au poids total de la phase huileuse. Par exemple, la polyoléfine à terminaison succinique commercialisée sous la dénomination Lubrizol 2724 par la société Lubrizol, à une concentration de 0,01% en poids par rapport au poids total de la phase huileuse, abaisse la tension interfaciale de 15 mN/m à l'interface d'une phase aqueuse constituée d'une solution aqueuse à 1% de MgSO4, et d'une phase huileuse comportant un mélange d'huiles (isohexadécane /polyisobutène hydrogéné/silicone volatile dans un rapport 8/6/4).

La concentration massique en tensioactif dérivé de polyoléfine va de préférence de 0,1 à 10%, et plus préférentiellement de 1 à 3% en matière active par raport au poids total de la composition. Ils peuvent être utilisés seul ou en mélange avec des agents amphiphiles pouvant stabiliser des émulsions eau-dans-huile (tensioactifs, polymères, particules).

Les composés 4,4-diarylbutadiènes conformes à l'invention sont choisis de préférence parmi ceux répondant a la formule (i) suivante : 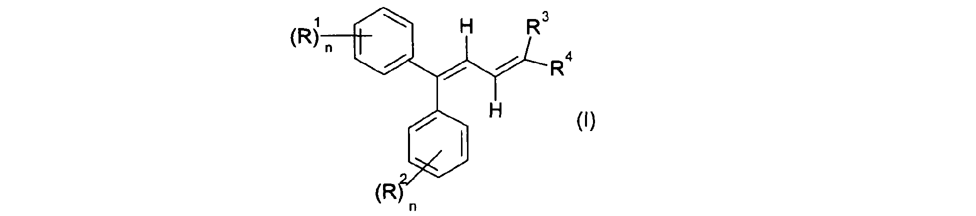 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C_{20,}; un radical alcényle en C₂- C₁₀ ; un radical alcoxy en C₁-C₁₂ ; un radical cycloalkyle en C₃-C₁₀ ; un radical cycloalcényle en C₃-C₁₀ ; un radical alcoxycarbonyle en C₁-C₂₀ ; un radical monoalkylamino en C₁-C₁₂ ; un radical dialkylamino en C₁-C₁₂ ; un aryle ; un hétéroaryle ou un substituant hydrosolubilisant choisi parmi un reste carboxylate, sulfonate ou un reste ammonium ;
- R³ désigne un groupe COOR⁵ ; COR⁵ ; CONR⁵R⁶ ; CN ; O=S(-R⁵)=O ; O=S(-OR⁵)=O ; R⁷O-P-(-OR⁸)=O; un radical alkyle en C₁-C₂₀ ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle en C₆-C₁₈ éventuellement substitué ; un hétéroaryle en C₃-C₇ éventuellement substitué;
- R⁴ désigne un groupe COOR⁶; COR⁶; CONR⁵R⁶ ; CN ; O=S(-R⁶)=O ; O=S(-OR⁶)=O ; R⁷O-P-(-OR⁸)=O; un radical alkyle en C₁-C₂₀ ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle en C₆-C₁₈ éventuellement substitué ; un hétéroaryle en C₃-C₇ éventuellement substitué;
- les radicaux R⁵ à R⁸, identiques ou différents, désignent hydrogène ; un radical alkyle en C₁-C₂₀ ; un radical alcényle en C₂-C₁₀ ; un radical cycloalkyle en C₃-C₁₀ ; un radical bicycloalkyle en C₇-C₁₀ ; un radical bicycloalcényle en C₃-C₁₀ ; un radical cycloalcényle en C₇-C₁₀ ; un aryle éventuellement substitué ; un hétéroaryle éventuellement substitué ;
- n varie de 1 à 3 ;
les radicaux R³ à R⁸ peuvent former entre eux avec les atomes de carbone auxquels ils sont liés, un noyau en C₅-C₆ pouvant être condensé.

Comme radicaux alkyle en C₁-C₂₀, on peut citer par exemple : méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 2,2-diméthylpropyle, 1-éthylpropyle, n-hexyle, 1,1-diméthylpropyle, 1,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme groupes alcènyle en C₂-C₁₀, on peut citer par exemple : éthènyle, n-propènyle, 1-méthyléthènyle, n-butènyle, 1-méthylpropènyle, 2-méthylpropènyle, 1,1-diméthyléthènyle, n-pentènyle, 1-méthylbutènyle, 2-méthylbutènyle, 3-méthylbutènyle, 2,2-diméthylpropènyle, 1-éthylpropènyle, n-hexènyle, 1,1-diméthylpropènyle, 1,2-diméthylpropènyle, 1-méthylpentènyle, 2-méthylpentènyle, 3-méthylpentènyle, 4-méthylpentènyle, 1,1-diméthylbutènyle, 1,2-diméthylbutènyle, 1,3-diméthylbutènyle, 2,2-diméthylbutènyle, 2,3-diméthylbutènyle, 3,3-diméthylbutènyle, 1-éthylbutènyle, 2-éthylbutènyle, 1,1,2-triméthytpropènyle, 1,2,2-triméthylpropènyle, 1-éthyl-1-méthylpropènyle, 1-éthyl-2-méthylpropènyle, n-heptènyle, n-octènyle, n-nonènyle, n-décènyle.

Comme radicaux alcoxy en C₁-C₁₂, on peut citer : méthoxy, n-propoxy, 1-méthylpropoxy, 1-méthyléthoxy, n-pentoxy, 3-méthylbutoxy, 2,2-diméthylpropoxy, 1-méthyl-1-éthylpropoxy, octoxy, éthoxy, n-propoxy, n-butoxy, 2-méthylpropoxy, 1,1-diméthylpropoxy, hexoxy, heptoxy, 2-éthylhexoxy.

Comme radicaux alcoxycarbonyle en C₁-C₂₀, on peut citer les esters des alcools en C₁-C₂₀.

Comme radicaux monoalkylamino ou dialkylamino en C₁-C₁₂, on peut citer ceux dont le ou les radicaux alkyle sont choisis parmi méthyle, n-propyle, 2-méthylpropyle, 1,1-diméthyléthyle, hexyle, heptyle, 2-éthylhexyle, isopropyle, 1-méthylpropyle, n-pentyle, 3-méthylbutyle, 2,2-diméthylpropyle, 1-méthyl-1-éthylpropyle, octyle.

Comme radicaux cycloalkyles en C₃-C₁₀, on peut citer par exemple : cyclopropyle, cyclobutyle, cyclopentyle, cyclohexyle, cycloheptyle, 1-méthylcyclopropyle, 1-éthylcyclopropyle, 1-propylcyclopropyle, 1-butylcyclopropyle, 1-pentylcyclopropyle, 1-méthyl-1-butylcyclopropyle, 1,2-diméthylcyclypropyle, 1-méthyl-2-éthylcyclopropyle, cyclooctyle, cyclononyle ou cyclodécyle.

Comme radicaux cycloalcènyles en C₃-C₁₀ ayant une ou plusieurs doubles liaisons, on peut citer : cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctétraènyle, cyclononènyle ou cyclodécènyle.

Les radicaux cycloalkyles ou cycloalcényles peuvent comporter un ou plusieurs substituants (de préférence de 1 à 3) choisis par exemple parmi les halogènes comme chlore, fluor ou brome ; cyano ; nitro ; amino ; C₁-C₄-alkylamino ; C₁-C₄ dialkylamino ; C₁-C₄alkyle ; C₁-C₄-alcoxy ; hydroxy ; ils peuvent également comporter de 1 à 3 hétéroatomes comme souffre, oxygène ou azote dont les valences libres peuvent être saturées par un hydrogène ou un radical alkyle en C₁-C₄.

Les groupes bicycloalkyles ou bicycloalcényles sont choisis par exemple parmi les terpènes bicycliques comme les dérivés de pinane, de bornane, de pinène ou de camphre ou d'adamantane.

Les groupes aryles sont de préférence choisis parmi les cycles phényle ou naphtyle, lesquels pouvant comporter un ou plusieurs substituants (de préférence de 1 à 3) chois par exemple parmi halogène comme chlore, fluor ou brome ; cyano ; nitro ; amino ; C₁-C₄-alkylamino ; C₁-C₄ dialkylamino ; C₁-C₄alkyle ; C₁-C₄-alcoxy ; hydroxy. On préfère plus particulièrement phényle, méthoxyphényle , naphtyle, thienyle.

Les groupes hétéroaryles comportent en général un ou plusieurs hétéroatomes choisis parmi souffre, oxygène ou azote.

Les groupes hydrosolubilisants sont par exemple des restes carboxy, sulfoxy et plus particulièrement leurs sels avec des cations physiologiquement acceptables comme les sels de métaux alcalins ou les sels de trialkylammonium comme les sels de tri(hydroxyalkyl)ammonium ou de 2-méthylpropan-1-ol-2-ammonium. On peut également citer les groupes ammonium comme les alkylammoniums et leurs formes salifiées avec des anions physiologiquement acceptables.

Les composés de formule (I) sont connus en eux-mêmes et leurs structures et leurs synthèses sont décrites dans ies demandes de brevet DE19755649 , EP916335, EP1133980 et EP1133981.

A titre d'exemple de composé de formule (I), on peut citer les composés suivants :

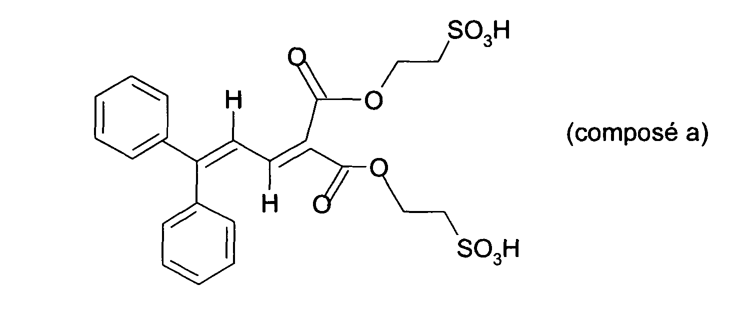

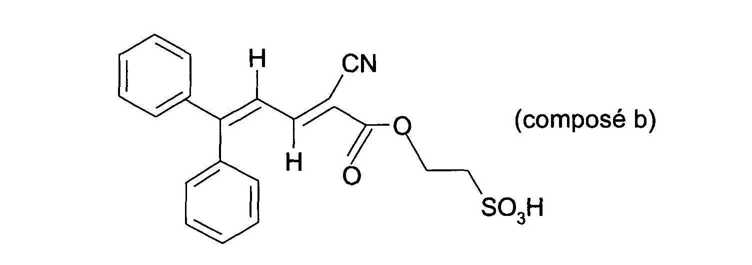

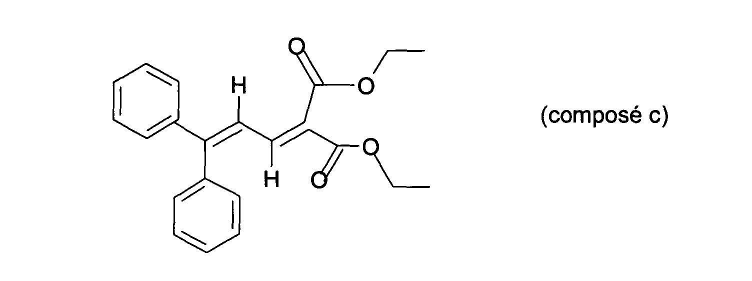

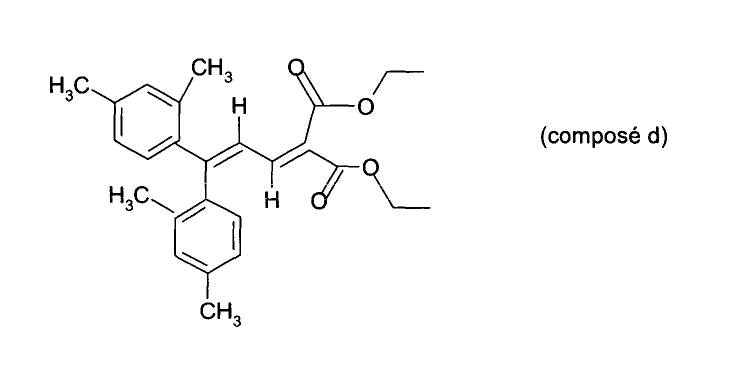

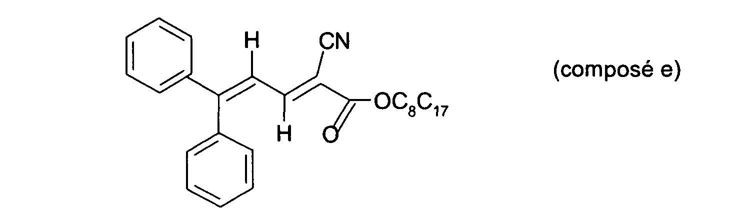

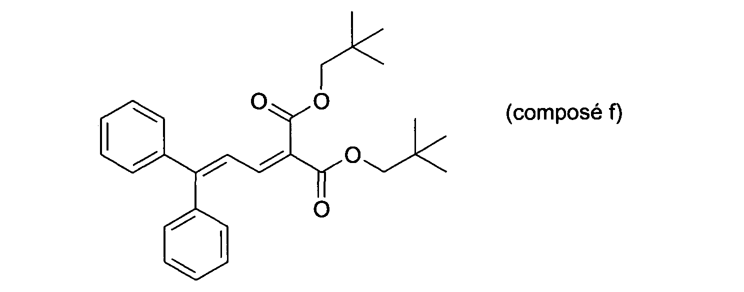

Les composés de formule (I) préférentiels sont ceux pour lesquels
- n = 1 ou 2 ;
- R¹ et R², identiques ou différents, désignent hydrogène, un radical alkyle en C₁-C₂₀,; un radical alcoxy en C₁-C₁₂ ; un radical monoalkylamino en C₁-C₁₂ ; un radical dialkylamino en C₁-C₁₂ ; un substituant hydrosolubilisant choisi parmi un groupe carboxylate, un groupe sulfonate ou un reste ammonium ;
- R³ désigne un groupe COOR⁵ ; COR⁵ ; CONR⁵R⁶; un radical alkyle en C₁-C₂₀ ; un radical cycloalkyle en C₃-C₁₀ ; un radical cycloalcényle en C₃-C₁₀ un radical bicycloalkyle en C₇-C₁₀ ; phényle, naphtyle ou thienyle éventuellement substitué ;
- R⁴ désigne un groupe COOR⁶ ; COR⁶ ; CONR⁵R⁶ ; un radical alkyle en C₁-C₂₀ ; un radical cycloalkyle en C₃-C₆ ; un radical cycloalcényle en C₃-C₁₀ ; un radical bicycloalkyle en C₇-C₁₀ ; phényle, naphtyle ou thienyle éventuellement substitué ;
- les radicaux R⁵ et R⁶, identiques ou différents, désignent hydrogène ; un radical alkyle en C₁-C₁₂ ; un radical cycloalkyle en C₃-C₁₀ ; un radical cycloalcényle en C₃-C₁₀ ; un radical bicycloalkyle en C₇-C₁₀ ; un radical bicycloalcényle en C₃-C₁₀ ; phényle ou naphtyle éventuellement substitué.
   Parmi ces composés, on préfère plus particulièrement ceux pour lesquels
- R¹ et R², identiques ou différents, désignent hydrogène, un radical alkyle en C₁-C₂₀ ; un radical alcoxy en C₁-C₂₀ ; un substituant hydrosolubilisant choisi parmi un groupe carboxylate, un groupe sulfonate ou un reste ammonium ;
- R³ désigne un groupe COOR⁵ ; COR⁵ ; CONR⁵R⁶ ;
- R⁴ désigne un groupe COOR⁶ ; COR⁶ ; CONR⁵R⁶ ;
- les radicaux R⁵ et R⁶, identiques ou différents, désignent hydrogène ; un radical alkyle en C₁-C₁₂ : un radical cycloalkyle en C₃-C₆ ; un radical cycloalcényle en C₃-C₁₀ ; un radical bicycloalkyle en C₇-C₁₀ ; un radical bicycloalcényle en C₃-C₁₀ ; phényle ou naphtyle éventuellement substitué.

Selon un mode particulièrement préféré, les composés de formule (I) sont choisis parmi ceux de formule (I') suivante : 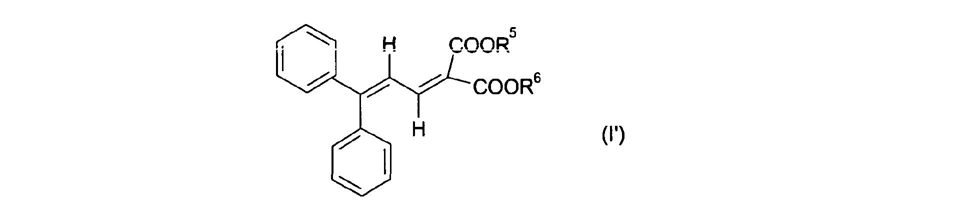 où les radicaux R⁵ et R⁶, identiques ou différents, désignent hydrogène ; un radical alkyle en C₁-C₂₀ ; un radical cycloalkyle en C₃-C₆ ; un radical cycloalcényle en C₃-C₁₀.

Parmi ces composés de formule (I'), on retient plus particulièrement le 1,1-dicarboxy-(2'2'-diméthyl-propyl)-4,4-diphénylbutadiène de structure :

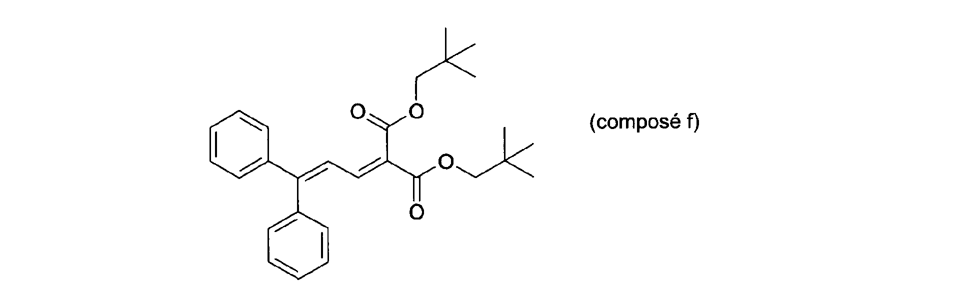

Une autre famille de 4,4-diarylbutadiène pouvant être utilisée dans les émulsions selon l'invention sont ceux répondant à la formule (II) suivante : 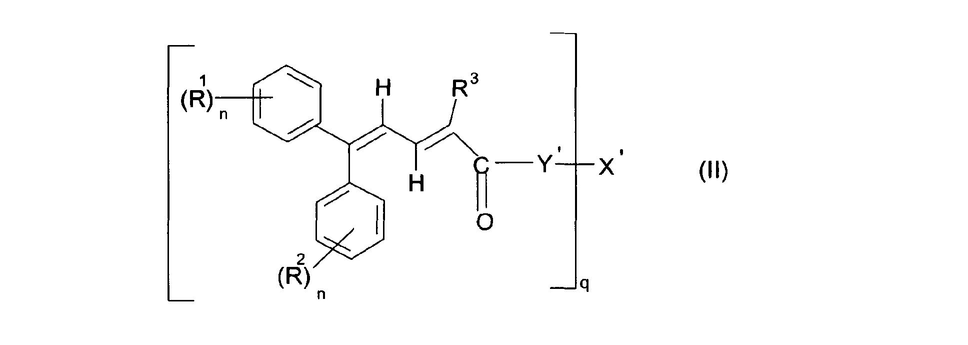 dans laquelle le système diène est de configuration Z,Z ; Z,E ; E,Z ou E,E ou des mélanges desdites configurations et où :
- R¹, R², R³ et n ont les mêmes significations indiquées dans la formule (I) précédente ;
- Y' désigne un groupe -O- ou -NR⁹-
- R⁹ désigne hydrogène ; un radical alkyle en C₁-C₂₀, linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; ; un radical bicycloalcényle en C₇-C₁₀ ; un aryle ; un hétéroaryle;
- X' désigne un reste de polyol en C₂-C₂₀ linéaire ou ramifié, aliphatique ou cycloaliphatique comprenant de 2 à10 groupes hydroxy et de valence q ; la chaîne carbonée dudit reste pouvant être interrompue par un ou plusieurs atomes de souffre ou d'oxygène ; un ou plusieurs groupes imines ; un ou plusieurs alkylimino en C₁-C₄ ;
- q varie de 2 à 10.

X' est un reste polyol en C₂-C₂₀ contenant de 2 à 10 groupes hydroxyles et notamment :

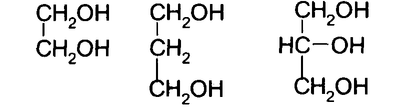

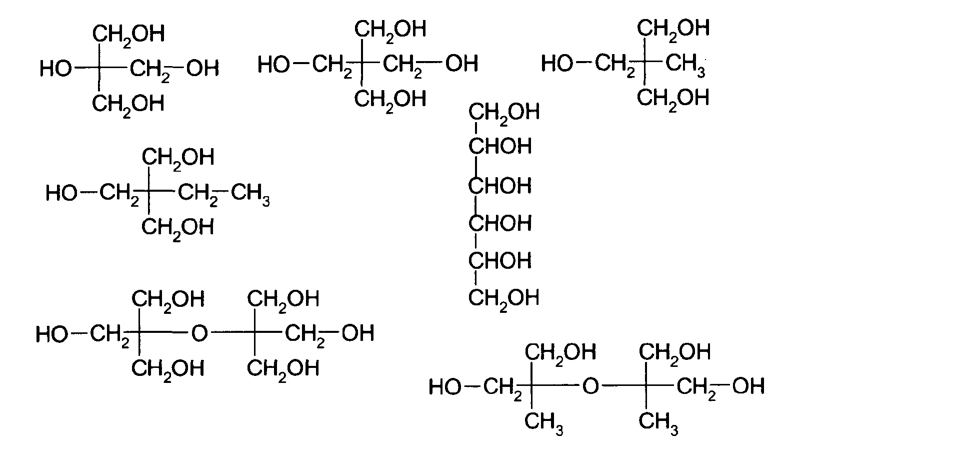

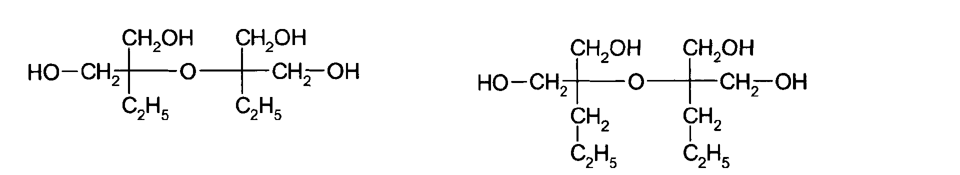

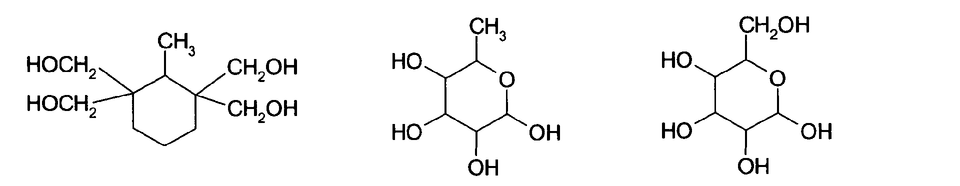

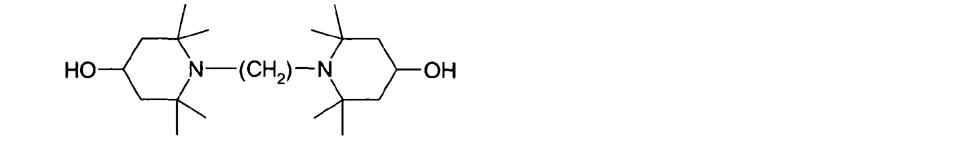

Les composés plus préférentiels de formule (II) sont ceux pour lesquels :
- R¹ et R², identiques ou différents, désignent hydrogène, un radical alkyle en C₁-C₁₂ ; un radicai aicoxy en C₁-C₈, un substituant hydrosolubilisant choisi parmi un groupe carboxylate, un groupe sulfonate ou un reste ammonium ;
- R³ désigne un groupe COOR⁵ ; CONR⁵R⁶ ; CN ; un radical cycloalkyle en C₃-C₁₀ ; un radical bicycloalkyle en C₇-C₁₀ ;
- R⁵ et R⁶ , identiques ou différents, désignent un radical alkyle en C₁-C₂₀, linéaire ou ramifié ; un radical cycloalkyle en C₃-C₁₀ ; un radical bicycloalkyle en C₇-C₁₀; naphtyle ou phényle éventuellement substitué ;
- X' désigne un reste de polyol comprenant de 2 à 6 groupes hydroxy et plus particulièrement de 2 à 4.

Les composés encore plus préférentiels de formule (II) sont ceux pour lesquels :
- X' désigne un reste d'éthanol ou de pentaerythrol.

Les composés de formule (II) encore plus particulièrement préférés sont choisis parmi

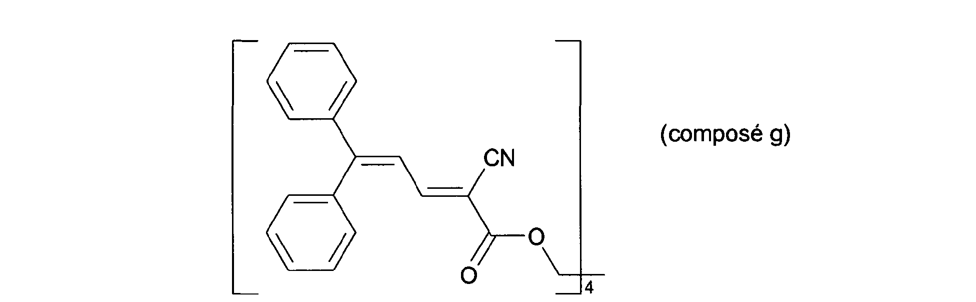

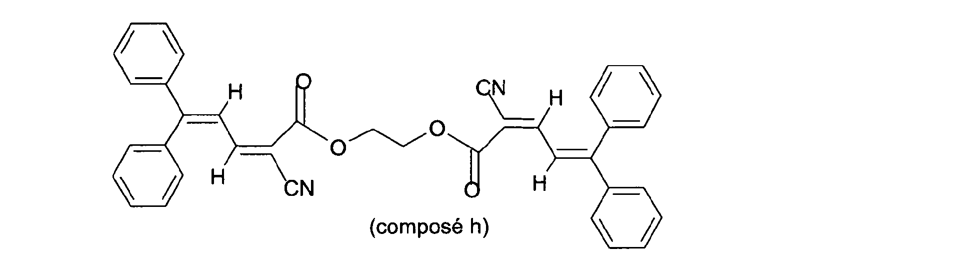

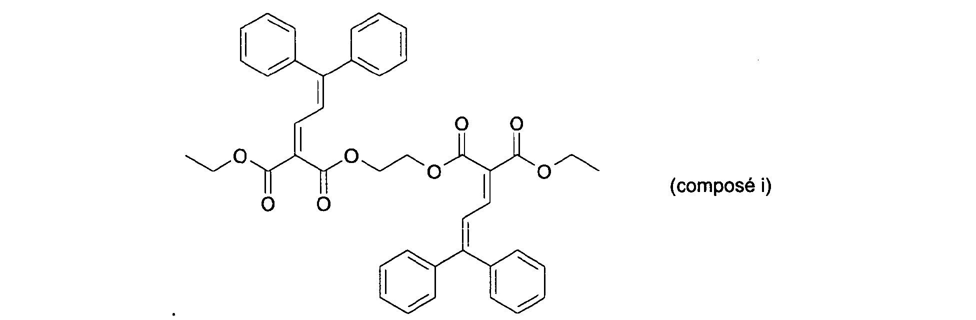

Les composés de formule (II) tels que définis ci-dessus sont connus en eux-mêmes et leurs structures et leurs synthèses sont décrites dans la demande de brevet EP-A-1008586.

Les composés 4,4-diarylbutadiène sont présents de préférence dans la composition dans des proportions allant de 0,1 % à 20% en poids, plus préférentiellement de 1 à 10% en poids par rapport au poids total de la composition.

La nature de la phase grasse rentrant dans la composition des émulsions selon l'invention n'est pas critique et elle peut ainsi être constituée par tous les composés qui sont déjà connus de façon générale comme convenant pour la fabrication d'émulsions de type eau dans huile. En particulier, ces composés peuvent être choisis, seuls ou en mélanges, parmi les différents corps gras, les huiles d'origine végétale, animale ou minérale, les cires naturelles ou synthétiques, et analogues.
Parmi les huiles pouvant rentrer dans la composition de la phase grasse, on peut notamment citer :
- les huiles minérales telles que l'huile de paraffine et l'huile de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore et l'huile de seigle,
- les huiles synthétiques telles que l'huile de purcellin, les esters comme par exemple le myristate de butyle, le myristate d'isopropyle, le myristate de cétyle, le palmitate d'isopropyle, l'adipate d'isopropyle, l'adipate d'éthylhéxyle, le stéarate de butyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle, le lanolate d'isocétyle, les isoparaffines et les poly-α-oléfines.

Comme autres huiles utilisables dans les émulsions selon l'invention, on peut encore citer les benzoates d'alcools gras en C12-C15 (Finsolv TN de FINETEX), les éthers, les dérivés lipophiles d'acide aminé tels que le N-lauroylsarcosinate d'isopropyl (Eldew SL-205 d'Ajinomoto), les alcools gras tels que l'alcool laurique, cétylique, myristique, stéarique, palmitique, oléique ainsi que le 2-octyldodécanol, les acétylglycérides, les octanoates et décanoates d'alcools et de polyalcools tels que ceux de glycol et de glycérol, les ricinoléates d'alcools et de polyalcools tels que ceux de cétyle, les triglycérides d'acides gras tels que les triglycérides caprylique/caprique, les triglycérides d'acides gras saturés en C10-C18, les huiles fluorées et perfluorées, la lanoline, la lanoline hydrogénée, la lanoline acétylée et enfin les huiles de silicones, volatiles ou non.

Bien entendu, la phase grasse peut également contenir un ou plusieurs adjuvants cosmétiques lipophiles classiques, comme par exemple des cires, des gélifiants lipophiles, des tensio-actifs, des particules organiques ou minérales, et notamment ceux qui sont déjà utilisés de manière habituelle dans la fabrication et l'obtention des compositions cosmétiques antisolaires.

De manière classique, la phase aqueuse dispersante a une teneur comprise entre 40% et 95% en poids. Elle peut être constituée par de l'eau, ou un mélange d'eau et d'alcool(s) polyhydrique(s) comme par exemple glycérol, propylèneglycol , butylène glycol et sorbitol, ou bien encore un mélange d'eau et d'alcool(s) inférieur(s) hydrosoluble(s) tels que éthanol, isopropanol ou butanol (solution hydroalcoolique).

Les émulsions conformes à l'invention peuvent comporter en plus d'autres filtres UV organiques ou inorganiques complémentaires actifs dans l'UVA et/ou l'UVB, hydrosolubles ou liposolubles ou bien insolubles dans les solvants cosmétiques couramment utilisés.

Les filtres organiques complémentaires sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US 5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 et leurs mélanges.

Comme exemples de filtres organiques actifs dans l'UV-A et/ou l'UV-B, on peut citer désignés ci-dessous sous leur nom INCI :
Dérivés de l'acide para-aminobenzoique :
   PABA,
   Ethyl PABA,
   Ethyl Dihydroxypropyl PABA,
   Ethylhexyl Diméthyl PABA vendu notamment sous le nom « ESCALOL 507 » par ISP,
   Glyceryl PABA,
   PEG-25 PABA vendu sous le nom « UVINUL P25 » par BASF,
Dérivés salicyliques:
   Homosalate vendu sous le nom « Eusolex HMS » par Rona/EM Industries,
   Ethylhexyl Salicylate vendu sous le nom « NEO HELIOPAN OS » par Haarmann et REIMER,
   Dipropyleneglycol Salicylate vendu sous le nom « DIPSAL » par SCHER,
   TEA Salicylate, vendu sous le nom « NEO HELIOPAN TS » par Haarmann et REIMER,
Dérivés du dibenzoylméthane :
   Butyl Methoxydibenzoylmethane vendu notamment sous le nom commercial « PARSOL 1789 » par HOFFMANN LAROCHE,
   Isopropyl Dibenzoylmethane,
Dérivés cinnamiques :
   Ethylhexyl Methoxycinnamate vendu notamment sous le nom commercial « PARSOL MCX » par HOFFMANN LAROCHE,
   Isopropyl Methoxy cinnamate,
   Isoamyl Methoxy cinnamate vendu sous le nom commercial « NEO HELIOPAN E 1000 » par HAARMANN et REIMER,
   Cinoxate,
   DEA Methoxycinnamate,
   - Diisopropyl Methylcinnamate,
   Glyceryl Ethylhexanoate Dimethoxycinnamate
Dérivés de β,β-diphénylacrytate :
   Octocrylene vendu notamment sous le nom commercial « UVINUL N539 » par BASF,
   Etocrylene, vendu notamment sous ie nom commerciai « UVINUL N35 » par BASF,
Dérivés de la benzophénone :
   Benzophenone-1 vendu sous le nom commercial « UVINUL 400 » par BASF,
   Benzophenone-2 vendu sous le nom commercial « UVINUL D50 » par BASF
   Benzophenone-3 ou Oxybenzone, vendu sous le nom commercial « UVINUL M40 » par BASF,
   Benzophenone-4 vendu sous le nom commercial « UVINUL MS40 » par BASF, Benzophenone-5
   Benzophenone-6 vendu sous le nom commercial « Helisorb 11 » par Norquay
   Benzophenone-8 vendu sous le nom commercial « Spectra-Sorb UV-24 » par American Cyanamid
   Benzophenone-9 vendu sous le nom commercial« UVINUL DS-49» par BASF, Benzophenone-12,
   le 2-(4-diéthylamino-2-hydroxybenzoyl)-benzoate de n-hexyle
Dérivés du benzylidène camphre :
   3-Benzylidene camphor fabriqué sous le nom « MEXORYL SD» par CHIMEX,
   4-Methylbenzylidene camphor vendu sous le nom « EUSOLEX 6300 » par MERCK , Benzylidene Camphor Sulfonic Acid fabriqué sous le nom « MEXORYL SL» par CHIMEX, Camphor Benzalkonium Methosulfate fabriqué sous le nom « MEXORYL SO » par CHIMEX,
   Terephthalylidene Dicamphor Sulfonic Acid fabriqué sous le nom « MEXORYL SX » par CHIMEX,
   Polyacrylamidomethyl Benzylidene Camphor fabriqué sous le nom « MESORYL SW » par CHIMEX,
Dérivés de benzimidazole :
   Phenylbenzimidazole Sulfonic Acid vendu notamment sous le nom commercial « EUSOLEX 232 » par MERCK,
   Disodium Phenyl Dibenzimidazole Tetra-sulfonate vendu sous le nom commercial commercial « NEO HELIOPAN AP » par Haarmann et REIMER,
Dérivés de triazine :
   Anisotriazine vendu sous le nom commercial «TINOSORB S » par CIBA SPECIALTY CHEMICALS
   Ethylhexyl triazone vendu notamment sous le nom commercial «UVINUL T150 » par BASF,
   Diethylhexyl Butamido Triazone vendu sous le nom commercial « UVASORB HEB » par SIGMA 3V
   la 2,4,6- tris-(4'amino-benzalmalonate de diisobutyle)-s-triazine.
Dérivés de benzotriazole :
   Drometrizole Trisiloxane vendu sous le nom « Silatrizole » par RHODIA CHIMIE ,
   Methylène bis-Benzotriazolyl Tetramethylbutylphénol, vendu sous forme solide sous le nom commercial « MIXXIM BB/100» par FAIRMOUNT CHEMICAL ou sous forme micronisé en dispersion aqueuse sous le nom commercial « TINOSORB M » par CIBA SPECIALTY CHEMICALS,
Dérivés anthraniliques :
   Menthyl anthranilate vendu sous le nom commercial commercial « NEO HELIOPAN MA » par Haarmann et REIMER,
Dérivés d'imidazolines :
   Ethylhexyl Dimethoxybenzylidene Dioxoimidazoline Propionate,
Dérivés de benzalmalonate :
   Polyorganosiloxane à fonctions benzalmalonate tel que le polysilicone-15 vendu sous la dénomination commerciale « PARSOL SLX » par HOFFMANN LAROCHE
Dérivés de benzoxazole :
   2,4-bis-[5-1 (diméthylpropyl)benzoxazol-2-yl-(4-phenyl)-imino]-6-(2-ethylhexyl)-imino-1,3,5-triazine vendu sous le nom Uvasorb K2A par Sigma 3V ;
et leurs mélanges.

Les filtres organiques plus particulièrement préférés sont choisis parmi les composés suivants :
Ethylhexyl Salicylate,
Ethylhexyl Methoxycinnamate
Octocrylene,
Butyl Methoxydibenzoylmethane
Phenylbenzimidazole Sulfonic Acid,
Benzophenone-3,
Benzophenone-4,
Benzophenone-5,
le 2-(4-diéthylamino-2-hydroxybenzoyl)-benzoate de n-hexyle 4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de diisobutyle)-s-triazine Anisotriazine,
Ethylhexyl triazone,
Diethylhexyl Butamido Triazone,
Methylène bis-Benzotriazolyl Tetramethylbutylphénol
Drometrizole Trisiloxane
Polysilicone 15
2,4-bis-[5-1(diméthylpropyl)benzoxazol-2-yl-(4-phenyl)-imino]-6-(2-ethylhexyl)-imino-1,3,5-triazine et leurs mélanges.

Les filtres complémentaires inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les filtres complémentaires selon l'invention sont généralement présents dans les compositions selon l'invention à une teneur allant de 0,1 % à 30 % en poids et de préférence de 0,5 à 15 % , en poids, par rapport au poids total de la composition.

Les compositions selon l'invention peuvent également contenir des agents de bronzage et/ou de brunissage artificiels de la peau (agents autobronzants).

Les agents autobronzants sont généralement choisis parmi les composés mono ou polycarbonylés tels que par exemple l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrites dans la demande de brevet FR 2 466 492 et WO 97/35842, la dihydroxyacétone (DHA), les dérivés de 4,4-dihydroxypyrazolin-5-ones telles que décrites dans la demande de brevet EP 903 342. On utilisera de préférence la DHA.

La DHA peut être utilisée sous forme libre et/ou encapsulée par exemple dans des vésicules lipidiques telle que des liposomes, notamment décrits dans la demande WO 97/25970.

Les agents autobronzants mono ou polycarbonylés sont généralement présents dans les compositions selon l'invention dans des proportions allant de 0,1 à 10% en poids par rapport au poids total de la composition, et de préférence de 0,2 à 8% en poids par rapport au poids total de la composition

Les compositions conformes à la présente invention peuvent comprendre en outre des adjuvants cosmétiques classiques notamment choisis parmi les solvants organiques, les épaississants ioniques ou non ioniques, les adoucissants, les humectants, les opacifiants, les stabilisants, les émollients, les silicones, les agents répulsifs contre les insectes, les parfums, les conservateurs, les tensioactifs, les charges, les actifs, les pigments, les polymères, les propulseurs, les agents alcalinisants ou acidifiants ou tout autre ingrédient habituellement utilisé dans le domaine cosmétique et/ou dermatologique.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Parmi les solvants organiques, on peut citer les alcools et polyols inférieurs.

Parmi les épaississants on peut citer les polymères acryliques réticulés comme les Carbomers fournis par Novéon, les polymères réticulés acrylates/C10-30 alkylacrylates du type Pemulen fournis par Novéon ou le polyacrylate-3 vendu sous le nom Viscophobe DB 1000 par Amerchol) ; les polymères dérivés de l'acide acrylamido 2-méthylpropane sulfonique (Hostacerin AMPS fourni par Clariant, Sépigel 305 fourni par Seppic), les polymères neutres synthétiques tels que la poly N-vinylpyrrolidone, les polysaccharides comme les gommes de guar, de xanthane et les dérivés cellulosiques modifiés ou non comme la gomme de guar hydroxypropylée, la méthylhydroxyéthylcellulose et l'hydroxypropylméthylcellulose.

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Les compositions cosmétiques selon l'invention peuvent par exemple être utilisées comme produit de soin et/ou de protection solaire pour le visage et/ou le corps de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517.

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans
des quantités allant de 15 à 50% en poids par rapport au poids total de la composition.

Les exemples concrets, mais nullement limitatifs, illustrant l'invention, vont maintenant être donnés.

### Exemples 1 et 2: Crèmes hydratantes et photoprotectrices

| **Ingrédients** | **Emulsion 1 (hors invention)** | **Emulsion 2 (invention)** |
|---|---|---|
| Hydroxyethyldiethonium polyisobutenyl triethylaminosuccinate (and) diethyl ethanolamine (Lubrizol 5603) | 1,92 | 1,92 |
| Isononanoate d'isononyle | 2,40 | 2,40 |
| Squalane | 3,20 | 3,20 |
| Dimethicone | 0,66 | 0,66 |
| Octyl-méthoxycinnamate | 3,00 | 3,00 |
| 1,1-dicarboxy-(2'2'-diméthyl-propyl)-4,4-diphénylbutadiène (composé f) | 0 | 1,32 |
| Glycérine | 5,00 | 5,00 |
| Acide téréphtalylidène 3,3'-dicamphosulfonique-10,10' | 1,32 | 0 |
| MgSO₄ | 2,00 | 2,00 |
| Triéthanolamine | 0,82 | 0 |
| Conservateur | 1,00 | 1,00 |
| Eau déminéralisée | qsp 100 | qsp 100 |

L'émulsion 1 ne peut se former. On observe 3 phases .

L'émulsion 2 est une crème homogène et stable, agréable à appliquer sur la peau et présentant de bonnes propriétés photoprotectrices.

Mode de préparation des émulsions : La phase aqueuse est progressivement incorporée sous agitation dans la phase huileuse comprenant les tensio-actifs. L'émulsion est réalisée à une température allant de 20°C à 60°C.

## Revendications

1. Emulsion eau-dans-huile comprenant au moins un tensio-actif polymérique constitué d'au moins une partie polaire et d'au moins une partie apolaire polyoléfinique, **caractérisée par le fait qu'**elle contient au moins un filtre UV-A organique du type 4,4-diarylbutadiène.

2. Emulsion selon la revendication 1, **caractérisée en ce que** ledit tensioactif polymérique comporte une partie apolaire polyoléfinique comprenant au moins 40 atomes de carbone et de préférence de 60 à 700 atomes de carbone.

3. Emulsion selon la revendication 2, **caractérisée en ce que** la partie apolaire polyoléfinique est choisie parmi les polymères et/ou les copolymères d'éthylène, de propylène, de 1-butène, d'isobutène, de 1-pentène, de 2-méthyl-1-butène, de 3-methyl-1-butène, de 1-héxène, de 1-heptène, de 1-octène, de 1-décène, de 1-undécène, de 1-dodécéne, de 1-tridécène, de 1-tetradécène, de 1-pentadécène, de 1-hexadécéne, de 1-heptadécène et de 1-octadécéne.

4. Emulsion selon l'une quelconque des revendications 1 à 3, **caractérisée en ce que** la partie polaire peut être anionique, cationique, non ionique, zwitterionique ou amphotère.

5. Emulsion selon l'une quelconque des revendications 1 à 4, **caractérisée en ce que** la partie polaire est constituée de polyalkylène glycols, de polyalkylène imines, d'acides ou de diacides carboxyliques, de leurs anhydrides ou dérivés, et leurs mélanges.

6. Composition selon l'une quelconque des revendications 1 à 5, **caractérisée en ce que** la partie polaire est choisie dans le groupe comprenant le polyoxyéthylène, l'acide ou l'anhydride succinique et leurs dérivés.

7. Emulsion selon l'une quelconque des revendications 1 à 6, **caractérisée en ce que** le tensio-actif polymérique est issu de la réaction entre un dérivé de polyoléfine et au moins un acide ou anhydride carboxylique choisi dans le groupe comprenant l'acide maléique ; l'anhydride maléique ; l'acide fumarique ; l'acide itaconique ; l'acide citraconique ; l'acide mésaconique ; l'acide aconitique ; leurs dérivés et leurs mélanges.

8. Emulsion selon l'une quelconque des revendications 1 à 7, **caractérisée en ce que** le tensio-actif polymérique est un polyisobutylène à terminaison succinique éventuellement modifiée.

9. Emulsion selon l'une quelconque des revendications 1 à 8, **caractérisée en ce que** le tensio-actif polymérique est le produit de la réaction de l'anhydride maléique avec le polyisobutylène.

10. Emulsion selon l'une quelconque des revendications 1 à 9, **caractérisée en ce que** ledit tensioactif polymérique abaisse la tension interfaciale d'au moins 10 mN/m à une concentration de 0,01% en poids par rapport au poids de phase huileuse.

11. Emulsion selon l'une quelconque des revendications 1 à 10, **caractérisée en ce que** la quantité de tensioactif polymérique va de 0,1 à 10%, et plus préférentiellement de 1 à 3% en poids de matière active par rapport au poids total de l'émulsion.

12. Emulsion selon l'une quelconque des revendications 1 à 11, où le filtre UV-A du type 4,4-diarylbutadiène répond à la formule (I) suivante : 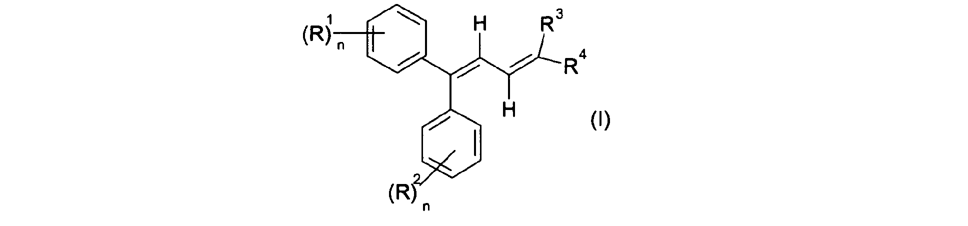 dans laquelle le système diène est de configuration Z,Z ; Z,E ; E,Z ou E,E ou des mélanges desdites configurations et où :
- R¹ et R², identiques ou différents, désignent hydrogène, un radical alkyle en C1-C20,; un radical alcényle en C₂- C₁₀ ; un radical alcoxy en C₁-C₁₂ ; un radical cycloalkyle en C₃-C₁₀ ; un radical cycloalcényle en C₃-C₁₀ ; un radical alcoxycarbonyle en C₁-C₂₀ ; un radical monoalkylamino en C₁-C₁₂ ; un radical dialkylamino en C₁-C₁₂ ; un aryle ; un hétéroaryle ou un substituant hydrosolubilisant choisi parmi un reste carboxylate, sulfonate ou un reste ammonium ;
- R³ désigne un groupe COOR⁵ ; COR⁵ ; CONR⁵R⁶; CN ; O=S(-R⁵)=O ; O=S(-OR⁵)=O ; R⁷O-P-(-OR⁸)=O; un radical alkyle en C₁-C₂₀ ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle en C₆-C₁₈ éventuellement substitué ; un hétéroaryle en C₃-C₇ éventuellement substitué;
- R⁴ désigne un groupe COOR⁶; COR⁶; CONR⁵R⁶ ; CN ; O=S(-R⁶)=O ; O=S(-OR⁶)=O ; R⁷O-P-(-OR⁸)=O; un radical alkyle en C₁-C₂₀ ; un radical alcényle en C₂- C₁₀ ; un radical cycloalkyle en C₃-C₁₀ ; un radical bicycloalkyle en C₇-C₁₀ ; un radical cycloalcényle en C₃-C₁₀; un radical bicycloalcényle en C₇-C₁₀ ; un aryle en C₆-C₁₈ éventuellement substitué ; un hétéroaryle en C₃-C₇ éventuellement substitué;
- les radicaux R⁵ à R⁸, identiques ou différents, désignent hydrogène ; un radical alkyle en C₁-C₂₀ ; un radical alcényle en C₂-C₁₀ ; un radical cycloalkyle en C₃-C₁₀ ; un radical bicycloalkyle en C₇-C₁₀ ; un radical bicycloalcényle en C₃-C₁₀ ; un radical cycloalcényle en C₇-C₁₀ ; un aryle éventuellement substitué ; un hétéroaryle éventuellement substitué ;
- n varie de 1 à 3 ;
les radicaux R³ à R⁸ peuvent former entre eux avec les atomes de carbone auxquels ils sont liés, un noyau en C₅-C₆ pouvant être condensé.

13. Emulsion selon la revendication 12, où le composé de formule (I) est chois parmi ceux pour lesquels
- n = 1 ou 2 ;
- R¹ et R², identiques ou différents, désignent hydrogène, un radical alkyle en C₁-C₂₀; un radical alcoxy en C₁-C₁₂; un radical monoalkylamino en C₁-C₁₂ ; un radical dialkylamino en C₁-C₁₂ ; un substituant hydrosolubilisant choisi parmi un groupe carboxylate, un groupe sulfonate ou un reste ammonium ;
- R³ désigne un groupe COOR⁵ ; COR⁵ ; CONR⁵R⁶ ; un radical alkyle en C₁-C₂₀ ; un radical cycloalkyle en C₃-C₁₀ ; un radical cycloalcényle en C₃-C₁₀ un radical bicycloalkyle en C₇-C₁₀ ; phényle, naphtyle ou thienyle éventuellement substitué ;
- R⁴ désigne un groupe COOR⁶ ; COR⁶; CONR⁵R⁶ ; un radical alkyle en C₁-C₂₀ ; un radical cycloalkyle en C₃-C₆ ; un radical cycloalcényle en C₃-C₁₀ ; un radical bicycloalkyle en C₇-C₁₀ ; phényle, naphtyle ou thienyle éventuellement substitué ;
- les radicaux R⁵ et R⁶, identiques ou différents, désignent hydrogène ; un radical alkyle en C₁-C₁₂ ; un radical cycloalkyle en C₃-C₁₀ ; un radical cycloalcényle en C₃-C₁₀ ; un radical bicycloalkyle en C₇-C₁₀ ; un radical bicycloalcényle en C₃-C₁₀ ; phényle ou naphtyle éventuellement substitué.

14. Emulsion selon la revendication 13, où le composé de formule (I) est chois parmi ceux pour lesquels
- R¹ et R², identiques ou différents, désignent hydrogène, un radical alkyle en C₁-C₂₀ ; un radical alcoxy en C₁-C₂₀ ; un substituant hydrosolubilisant choisi parmi un groupe carboxylate, un groupe sulfonate ou un reste ammonium ;
- R³ désigne un groupe COOR⁵ ; COR⁵ ; CONR⁵R⁶ ;
- R⁴ désigne un groupe COOR⁶ ; COR⁶ ; CONR⁵R⁶ ;
- les radicaux R⁵ et R⁶, identiques ou différents, désignent hydrogène ; un radical alkyle en C₁-C₁₂ ; un radical cycloalkyle en C₃-C₆ ; un radical cycloalcényle en C₃-C₁₀ ; un radical bicycloalkyle en C₇-C₁₀ ; un radical bicycloalcényle en C₃-C₁₀ ; phényle ou naphtyle éventuellement substitué.

15. Emulsion selon la revendication 14, où le composé de formule (I) est choisi parmi ceux de formule (I') suivante : 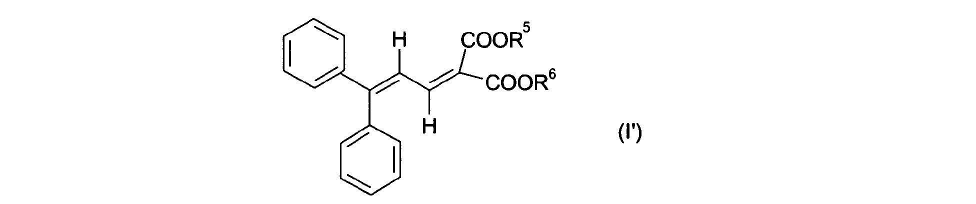 où les radicaux R⁵ et R⁶, identiques ou différents, désignent hydrogène ; un radical alkyle en C₁-C₂₀ ; un radical cycloalkyle en C₃-C₆ ; un radical cycloalcényle en C₃-C₁₀.

16. Emulsion selon la revendication 15, où le composé de formule (I') est le 1,1-dicarboxy-(2'2'-diméthyl-propyl)-4,4-diphénylbutadiène de structure :

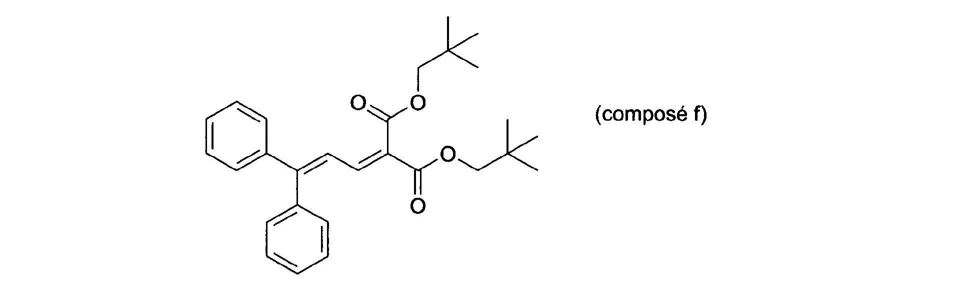

17. Emulsion selon l'une quelconque des revendications 1 à 11, où le filtre UV-A du type 4,4-diarylbutadiène répond à la formule (II) suivante : 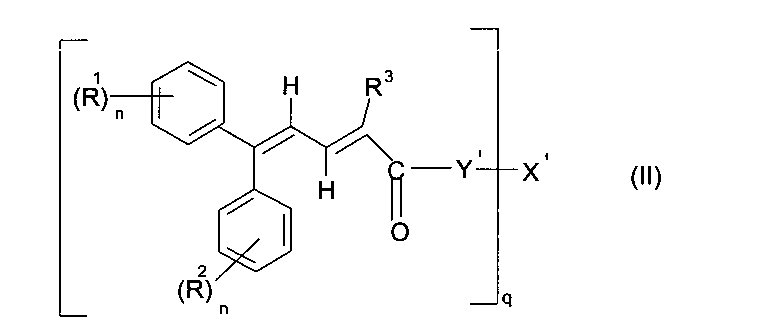 dans laquelle le système diène est de configuration Z,Z ; Z,E ; E,Z ou E,E ou des mélanges desdites configurations et où :
- R¹, R², R³ et n ont les mêmes significations indiquées dans la formule (I) telle que définie dans la revendication 12;
- Y' désigne un groupe -O- ou -NR⁹-
- R⁹ désigne hydrogène ; un radical alkyle en C₁-C₂₀, linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle ; un hétéroaryle;
- X' désigne un reste de polyol en C₂-C₂₀ linéaire ou ramifié, aliphatique ou cycloaliphatique comprenant de 2 à10 groupes hydroxy et de valence q ; la chaîne carbonée dudit reste pouvant être interrompue par un ou plusieurs atomes de souffre ou d'oxygène ; un ou plusieurs groupes imines ; un ou plusieurs alkylimino en C₁-C₄ ;
- q varie de 2 à 10.

18. Emulsion selon la revendication 17, où le composé de formule (II) est choisi parmi ceux pour lesquels :
- R¹ et R², identiques ou différents, désignent hydrogène, un radical alkyle en C₁-C₁₂ ; un radical alcoxy en C₁-C₈ ; un substituant hydrosolubilisant choisi parmi un groupe carboxylate, un groupe sulfonate ou un reste ammonium ;
- R³ désigne un groupe COOR⁵ ; CONR⁵R⁶ ; CN ; un radical cycloalkyle en C₃-C₁₀ ; un radical bicycloalkyle en C₇-C₁₀ ;
- R⁵ et R⁶ , identiques ou différents, désignent un radical alkyle en C₁-C₂₀, linéaire ou ramifié ; un radical cycloalkyle en C₃-C₁₀ ; un radical bicycloalkyle en C₇-C₁₀; naphtyle ou phényle éventuellement substitué ;
- X' désigne un reste de polyol en C₂-C₂₀ comprenant de 2 à 6 groupes hydroxy et plus particulièrement de 2 à 4.

19. Emulsion selon la revendication 18, où le composé de formule (II) est choisi parmi ceux pour lesquels X' désigne un reste d'éthanol ou de pentaerythritol.

20. Emulsion selon la revendication 19, où le composé de formule (II) est choisi parmi les composés suivants :

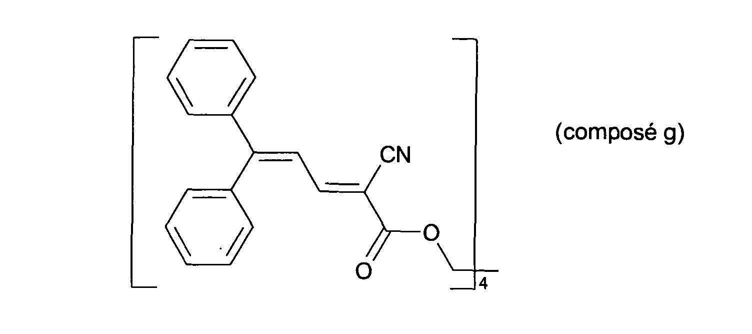

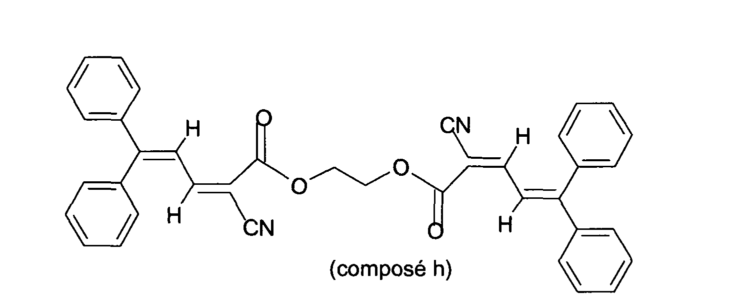

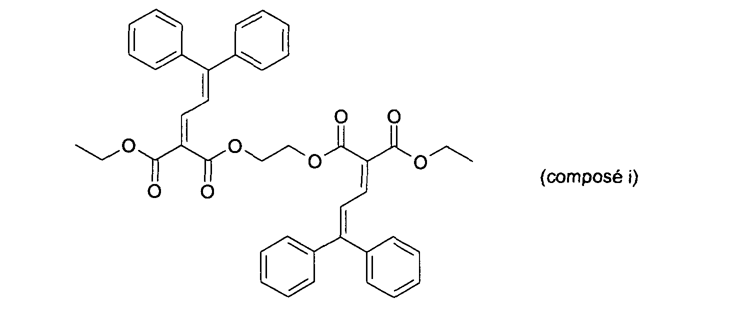

21. Emulsion selon l'une quelconque des revendications 1 à 20, **caractérisée en ce que** le ou les composés 4,4-diarylbutadiène sont présents dans des proportions allant de 0,1 % à 20% en poids, plus préférentiellement de 1 à 10% en poids par rapport au poids total de l'émulsion.

22. Emulsion selon l'une quelconque des revendications 1 à 21, **caractérisée en ce qu'**elle contient en outre au moins un filtre solaire organique ou inorganique complémentaire actif dans l'UV-A et/ou l'UV-B, hydrosolubles, liposolubles ou bien insolubles dans les solvants cosmétiques couramment utilisés.

23. Emulsion selon la revendication 22, ou les filtres organiques complémentaires sont choisis parmi les anthranilates ; les dérivés cinnamiques ; les dérivés de dibenzoylméthane ; les dérivés salicyliques, les dérivés du camphre ; les dérivés de triazine ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène et leurs mélanges.

24. Emulsion selon la revendication 23, ou les filtres organiques complémentaires sont choisis parmi
Ethylhexyl Salicylate,
Ethylhexyl Methoxycinnamate
Octocrylene,
Butyl Methoxydibenzoylmethane
Phenylbenzimidazole Sulfonic Acid,
Benzophenone-3,
Benzophenone-4,
Benzophenone-5,
le 2-(4-diéthylamino-2-hydroxybenzoyl)-benzoate de n-hexyle
4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de diisobutyle)-s-triazine Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
2,4-bis-[5-1 (diméthylpropyl)benzoxazol-2-yl-(4-phenyl)-imino]-6-(2-ethylhexyl)-imino-1,3,5-triazine
et leurs mélanges.

25. Emulsion selon la revendication 22, où les filtres complémentaires inorganiques sont choisis parmi des pigments ou des nanopigments d'oxydes métalliques, enrobés ou non.

26. Emulsion selon la revendication 25, où les filtres complémentaires inorganiques sont des nanopigments d'oxyde de titane, amorphe ou cristallisé, sous forme rutile et/ou anatase, de fer, de zinc, de zirconium ou de cérium

27. Emulsion selon l'une quelconque des revendications 1 à 26, **caractérisée en ce qu'**elle contient en outre au moins un agent de bronzage et/ou de brunissage artificiel de la peau.

28. Emulsion selon l'une quelconque des revendications 1 à 27, **caractérisée en ce qu'**elle contient en outre au moins un adjuvant cosmétique choisi parmi les solvants organiques, les épaississants ioniques ou non ioniques, les adoucissants, les humectants, les opacifiants, les stabilisants, les émollients, les silicones, les agents répulsifs contre les insectes, les parfums, les conservateurs, les tensioactifs, les charges, les actifs, les pigments, les polymères, les propulseurs, les agents alcalinisants ou acidifiants ou tout autre ingrédient habituellement utilisé dans le domaine cosmétique et/ou dermatologique.

29. Utilisation d'une émulsion définie à l'une quelconque des revendications 1 à 28 pour la fabrication d'une composition cosmétique ou dermatologique pour la protection de la peau et/ou des cheveux contre le rayonnement ultraviolet, en particulier le rayonnement solaire.
